# EUROPEAN PATENT APPLICATION

(11) **EP 4 800 131 A1**
(43) Date of publication of application: **02.09.2026**
(21) Application number: 24881865.0
(22) Date of filing: 18.10.2024
(51) Int. Cl.: C22B 3/18, C22B 15/00, H05K 3/22

(54) **ORGANIC BIOREAGENT, PRODUCTION METHOD, USE OF THE BIOREAGENT, AND LEACHING PROCESS TO RECOVER METALS**

(30) Priority: 23.10.2023 CL 202303161
(71) Applicant: Centro de Ingeniería, Investigación e Innovación SpA, San Joaquín. Santiago (CL)
(72) Inventor: MALVERDE FERNÁNDEZ, Sebastián Enrique, 2405B, San Joaquín. Santiago (CL); VELÁSQUEZ YÉVENES, Lilian de Lourdes, 2405B, San Joaquín. Santiago (CL)
(74) Representative: Temiño Ceniceros, Ignacio
(86) International application number: PCT/IB2024/060276
(87) International publication number: WO 2025/088455

(57) **Abstract**

The present invention discloses an organic "BioReagent" or leaching solution, a process for obtaining said BioReagent, and the use of the organic BioReagent in leaching processes for recovering precious metals and copper from Waste Electrical and Electronic Equipment (WEEE). The organic BioReagent of the present invention is produced from waste generated by the wine-making, forestry and/or agricultural industries. The BioReagent may further comprise microorganisms that promote the oxidation of oxidizing agents such as iron, thereby enhancing the dissolution of certain metallic elements to be recovered. The organic BioReagent of the present invention enables the selective dissolution of metallic elements present in size-reduced solid materials such as WEEE, while leaving more noble metals in their solid and liberated form.

## Description

### TECHNICAL FIELD

The present invention discloses an organic "BioReagent", also referred to herein as a "leaching solution", a method for obtaining said organic BioReagent, and the use of the organic BioReagent in leaching processes for recovering precious metals and copper from Waste Electrical and Electronic Equipment (WEEE). The organic BioReagent of the present invention is produced from waste generated by the wine-making, forestry and/or agricultural industries.

The winemaking process generates a large amount of solid waste, which has a high potential value, since grape stalks, skins, and seeds are rich in polyphenols, tannins, and organic acids, which act as natural antioxidants. In addition, the BioReagent may contain microorganisms that promote the oxidation of iron species, thereby enhancing the dissolution of certain metallic elements to be recovered.

Using the organic BioReagent of the present invention, a selective dissolution of metallic elements present in size-reduced solid materials, such as Waste Electrical and Electronic Equipment (WEEE), is achieved, including computing equipment, printed circuit boards (PCBs) from computers and mobile phones, monitors, displays, photovoltaic panels, and the like, while other more noble metals remain in a solid and liberated form.

### BACKGROUND

Large companies in the wine-making, forestry and agricultural sectors generate considerable amounts of waste through their forestry and winemaking activities, disposing of or managing the final residues through processes that are not environmentally friendly. Part of the biomass generated as waste from forestry production activities, such as logging, thinning, and pruning, as well as from wood processing of coniferous or myrtaceous species, is subjected to controlled burning in order to prevent fires or accidents. On the other hand, wine production generates grape residues which, in most cases, are disposed of on land or in the environment, resulting in natural decomposition through bacterial activity and fermentation processes of residual sugars, which generate alcohols and release CO₂. Such processes contribute to soil and water contamination due to increased acidity, as well as to the emission of greenhouse gases into the atmosphere. It is estimated that the annual generation in Chile of waste derived from both industrial activities and having potential technological application to the present invention ranges between 1 and 2 million tons.

Mining is an activity often regarded as controversial, however, its role has undoubtedly been fundamental to modern society. Its contributions range from transportation and infrastructure to energy generation and information technologies. Nevertheless, due to issues related to metal price fluctuations, resource scarcity, and the access to and availability of mineral sources, improving the recovery of metals from secondary resources has become an evident necessity. This situation places increasing pressure on the extraction of metals considered non-renewable resources, many of which are showing signs of depletion. It is estimated that the economically exploitable reserves of Au, Ag, and Cu may be exhausted within the next fifty years. Recent studies suggest that the mining industry is not adequately prepared to meet the growing demand for metals required by the electric vehicle industry boom, and therefore new sources of such metals are expected to originate from electrical and electronic waste. On the other hand, the lack of proper waste management may result in severe damage to human health and the environment, thereby jeopardizing sustainable development. For this reason, the safe disposal of such waste, together with the recovery of the metals contained therein, has become an urgent need.

Worldwide, the generation of another type of waste, namely electrical and electronic waste (WEEE), is increasing on an annual basis. WEEE generated at the global level is valorized through the recovery of the metallic elements contained therein, and it is estimated that up to USD 57 billion per year could be recovered. It is further estimated that approximately 36% of this value is contributed by the gold, silver, and copper content. However, despite the significant environmental impact of such waste and the substantial business opportunity associated with the metals present therein, only a small fraction of the WEEE generated worldwide is currently collected.

These wastes contain valuable metals such as Au, Ag, Pt, Pd, and Cu, among others, and it is estimated that certain WEEE streams may contain up to one hundred times more gold per ton than primary gold ores. Globally, more than 50 million tons of WEEE are generated per year, growing at a rate up to four times higher than other types of waste, due to the strong correlation between per capita GDP and the consumption of electrical and electronic products, resulting in an estimated loss of approximately USD 62.5 billion that is disposed of in landfills due to the lack of proper management of this type of waste.

Recycling and valorization are considered to be among the most sustainable options for the management of WEEE.

This approach is consistent with the principles underlying what is currently known as "Urban Mining", a concept based on the notion of urban mines, according to which the metals contained in WEEE are significantly more abundant than those present in primary ores. In this context, it has been reported that certain WEEE streams may contain up to ten times higher metal concentrations than conventional ores, and that up to one hundred times more gold may be found in one ton of WEEE than in one ton of mined ore. In Chile, the generation of WEEE is expected to increase by approximately 45% by 2027. Urban Mining has emerged with the objective of assigning value to materials that are currently underutilized, while preventing millions of WEEE items from being disposed of in landfills or illegal dumping sites. However, processing this type of waste is not a trivial task, which has driven increasing research efforts toward the development of sustainable and environmentally friendly processing routes for such materials. This aspect is of particular relevance, as proper treatment would not only contribute to environmental protection, but would also enable significant valorization of the waste, thereby allowing the economic self-sustainability of waste treatment processes.

Worldwide, conventional technology, namely pyrometallurgy, has been used in practice for many years for the recovery of precious metals from waste electronic equipment. However, due to the intrinsic limitations of conventional pyrometallurgical processes, such as high energy consumption, environmental hazards associated with gas emissions, the need for additional processing steps, lack of selectivity, and high capital costs, hydrometallurgical methods represent a significant opportunity for the processing of WEEE.

WEEE leaching processes are generally based on the use of inorganic acids combined with strong oxidizing agents at extreme concentrations, employing methodologies that are poorly suited for industrial application. Base metals such as Zn, Sn, Fe, and Al can be dissolved in dilute acids; however, Cu and precious metals such as Au and Pd are only significantly dissolved in strong acids in the presence of oxidizing agents, such as air/O₂, H₂O₂, Cl₂, Cu²⁺, Fe³⁺, and the like. In most cases, metals such as Ag and Pb pose particular challenges for effective acid leaching due to the wide range of insoluble salts formed by these metals.

Generally, dilute sulfuric acid (H₂SO₄) at atmospheric pressure (1 atm) is not capable of leaching copper to a significant extent. For example, using 1 M H₂SO₄, only approximately 8.8% of Cu is leached at ambient temperature after 96 hours (U. Jadhav, 2015). However, when 1 M H₂SO₄ is applied under pressure (2 MPa) and at elevated temperature (approximately 120 °C), nearly complete leaching of Cu, as well as Ni, Zn, and Fe, can be achieved (B. Altansukh, 2016). Aqua regia, as a traditional leaching agent, is capable of non-selectively and aggressively leaching various types of base and precious metals, for example Cu, Pb, Zn, Ni, and Au (L. Zhang, 2016). It has been reported that acidic solutions can effectively leach many base metals from WEEE under relatively moderate conditions, whereas higher requirements in terms of leaching time, temperature, pressure, and acid and oxidant concentrations are necessary for the leaching of precious metals. It is generally agreed among researchers that hydrometallurgical routes represent an environmentally friendly and cost-effective approach for the treatment of WEEE.

In the state of the art, for example, document US 9,215,813 discloses recycling processes for printed circuit boards using compositions described as environmentally friendly, wherein electronic components, precious metals, and common metals can be collected for reuse and recycling. Said document discloses a process for treating WEEE and recovering, among other metals, precious metals, and describes the use of oxidizing agents and passivating agents in its various stages. Additionally, document WO 2012/024603 discloses recycling processes for electronic components extracted from printed circuit boards, in which precious metals and base metals are extracted from the electronic components using aqua regia, nitric acid (HNO₃), hydrochloric acid, hydrogen peroxide, ammonium chloride, and diethylene glycol monobenzyl ether (DEGBE) at a concentration of 25% v/v. Such reagents are associated with acute and chronic toxicity and with the emission of toxic vapors or gases; therefore, the disclosed process involves the use of substances that present environmental and health-related concerns.

At least gold, silver, and copper ions can be extracted from electronic components and reduced to their respective metals using the processes and compositions described in said documents.

However, none of the cited documents discloses an organic BioReagent, nor a method for obtaining and using the same, which operates with low concentrations of oxidizing agents or strong acids, under moderate temperature conditions and at atmospheric pressure (1 atm), for the recovery of copper and precious metals from shredded WEEE, as disclosed in the present patent application.

### DESCRIPTION OF THE FIGURES

Figure 1 shows the evolution of pH in Tests E6, E16, E17, E20, and E36 during organic waste processing (group D1).
Figure 2 shows the evolution of electrical conductivity in Tests E6, E16, E17, E20, and E36 during organic waste processing (group D1).
Figure 3 shows the evolution of pH during organic waste processing (group D2).
Figure 4 shows the evolution of electrical conductivity during organic waste processing (group D2).
Figure 5 shows the gold, silver, and copper grades of the WEEE groups.
Figure 6 shows the copper recovery kinetics for sample RA4.
Figure 7 shows the copper recovery kinetics during the leaching of sample RA4, corresponding to the evaluation of the BioReagent.
Figure 8 shows the evolution of pH during the leaching of inorganic sample RA4.
Figure 9 shows the evolution of redox potential (Eh) during the leaching of inorganic sample RA4.
Figure 10 shows the copper recovery kinetics during the leaching of inorganic sample RA5.
Figure 11 shows the recovery of Au and Ag during the leaching of inorganic sample RA4, corresponding to a fine solid product below 600 µm, and to the evaluation of BioReagents B 1.0 and B 2.0 in the processing of PCB-type WEEE.

### DESCRIPTION OF THE INVENTION

The present invention discloses an organic BioReagent (leaching solution), a method for obtaining said organic BioReagent, uses of the organic BioReagent, and a method for using the organic BioReagent to recover precious metals and copper from Waste Electrical and Electronic Equipment (WEEE).

### Description of the BioReagent

The BioReagent of the present invention comprises:
i. at least one of the following acids derived from organic residues obtained from waste generated by the wine-making, forestry and/or agricultural industries, such as: tartaric acid at a concentration ranging from 0 to 15 g/L, malic acid at a concentration ranging from 0 to 5.5 g/L, acetic acid at a concentration ranging from 0 to 6.5 g/L, citric acid at a concentration ranging from 0 to 5.0 g/L, and polyphenols at a concentration ranging from 0 to 20 g/L.
ii. a continental aqueous medium or a saline medium.
   In the present invention, the aqueous medium may be continental water, also referred to as aquifer water or water from natural watercourses, distilled water, non-desalinated seawater, or saline discard streams derived from lithium production processes or from seawater desalination processes. The latter may reach chloride concentrations ranging between 45 and 85 g/L, originating from sodium salts. Additionally, the discard salts may also include other sources of chloride ions, such as salts generated in lithium production processes, including bischofite, halite in solid form, and halite in liquid form. Furthermore, such discard salts typically contain elements such as Li, Mg, Ca, Na, K, B, and SO₄²⁻. The aqueous medium contains a chloride concentration ranging from 0 to 90 g/L.
iii. ferrous ions at a concentration ranging from 0.1 to 10 g/L.
iv. sulfuric acid at a concentration ranging from 0.1 to 25 g/L.
v. cupric ions at a concentration ranging from 0 to 10 g/L.
vi. an inoculum of mesophilic or thermophilic microorganisms (bacteria or archaea) at a concentration ranging from 0 to 150 × 10⁶ cells/mL, wherein the sulfur-oxidizing and/or iron-oxidizing bioleaching microorganisms are selected from Ferroplasma acidiphilum, Leptospirillum ferrooxidans, Acidianus infernus, Acidithiobacillus ferrooxidans, and Acidithiobacillus thiooxidans.

### Method for Obtaining the BioReagent or Leaching Solution

The organic BioReagent of the present invention is produced from residues or waste generated by the wine-making, forestry and/or agricultural industries that generate lignocellulosic biomass of coniferous or myrtaceous origin (e.g., *Eucalyptus*, pine), Vitaceae (e.g., grape pomace), and Rosaceae (e.g., apple peels, peach residues, or other fruit waste).

The method for producing the BioReagent or leaching solution comprises the following steps:
a) obtaining the organic residue and reducing its particle size to less than 6 inches (15.24 cm);
b) loading the size-reduced organic residue from step (a) at a concentration ranging from 0.1 to 300 g/L into a closed temperature-controlled bath at atmospheric pressure (1 atm) containing an aqueous medium at a temperature ranging from 20 to 95 °C, homogenizing and aerating the mixture for a period ranging from 0.5 to 6 hours to produce an enriched organic solution.

A temperature-controlled bath is used, or in general any heat exchange system capable of maintaining a controlled temperature during the process. The process is carried out in a closed system to prevent water evaporation.

The aqueous medium may be continental water, distilled water, seawater, or saline discard streams.

The difference between these media lies in the natural contribution of catalytically species to the process, such as chloride ions. Thus, seawater typically provides between 15 and 25 g/L of Cl⁻, saline discard streams provide between 50 and 70 g/L of Cl⁻, and continental or distilled water provides approximately 0 g/L of Cl⁻. The latter media therefore require the artificial incorporation of chloride species, for example from halite, saline discard streams, or by-products of lithium production.

The type of organic material may vary in origin depending on the application, as may mixtures thereof. The concentration of the pure organic material or mixtures thereof may range from 0.1 to 500 g/L.

c) stopping step (b) by allowing the temperature to decrease, thereby obtaining a slurry comprising a liquid phase and organic residue.

The temperature-controlled system is turned off, and the enriched organic solution is allowed to cool down to ambient temperature. The objective of this step is to prevent or reduce evaporation of the solutions prior to their subsequent discharge.

d) discharging the slurry obtained in step (c) and performing a solid-liquid separation once the medium has cooled to below 50 °C, by filtration until drainage of the solution ceases, thereby obtaining the loaded solution to be used for preparing the BioReagent, while the solid fraction is directed to composting.

The filtration step may be selected from pressure filtration, vacuum filtration, membrane filtration, filter press, tangential filtration, centrifugal filtration, or gravity filtration, at a maximum pressure of 120 psi (827.4 kPa), recovering on average approximately 95% of the loaded process solution from the organic residues.

e) incorporating into the enriched solution from step (d) chemical agents functioning as catalysts, selected from:
- chloride ions derived from pure salts, such as halite, or from discard salts, at a concentration ranging from 0 to 90 g/L;
- ferrous ions at a concentration ranging from 0.1 to 10 g/L;
- sulfuric acid at a concentration ranging from 0.1 to 25 g/L;
- cupric ions at a concentration ranging from 0 to 10 g/L; and
- an inoculum of sulfur-oxidizing and/or iron-oxidizing microorganisms at a concentration ranging from 0 to 150 × 10⁶ cells/mL.

The sulfur-oxidizing and/or iron-oxidizing bioleaching microorganisms are selected from Ferroplasma acidiphilum, Leptospirillum ferrooxidans, Acidianus infernus, Acidithiobacillus ferrooxidans, and Acidithiobacillus thiooxidans.

f) homogenizing the solution from step (e) by means of mechanical agitation in a vertically oriented vessel and/or by vibration until homogeneity is achieved, such that dissolved solids are uniformly distributed and concentration gradients are eliminated, thereby obtaining the BioReagent or leaching solution.

### Use of the BioReagent or Leaching Solution

In another embodiment of the invention, the organic BioReagent or leaching solution obtained by the previously described process is used in a hydrometallurgical process comprising agitated leaching of minerals, metal concentrates, mining tailings, spent ore residues, metallurgical slags, and Waste Electrical and Electronic Equipment (WEEE).

In a more specific embodiment of the invention, the organic BioReagent or leaching solution obtained by the previously described process is used for recovering metals such as gold, silver, palladium, and copper from Waste Electrical and Electronic Equipment (WEEE).

WEEE may include, without limitation, computing equipment, printed circuit boards (PCBs) from computers and mobile phones, televisions and monitors, displays, photovoltaic panels, solar energy systems, desktop and laptop computers, tablets and similar electronic devices, lighting devices, kitchen appliances such as microwaves and toasters, washing machines and dryers, refrigerators and freezers, air conditioning and heating units, audio equipment and sound systems, cameras and camcorders, video game consoles and accessories, storage devices such as hard drives and USB memory devices, printers and scanners, electronic exercise equipment, medical electronic devices, electrical and electronic tools, surveillance and security equipment, communication equipment such as routers and modems, electronic toys and entertainment devices, watches and wearable devices, electronic office equipment such as photocopiers and fax machines, electronic lighting equipment such as LED lamps, industrial control and automation equipment, general consumer electronics, large rechargeable batteries such as those used in electric vehicles, cryptocurrency mining equipment such as ASICs and mining rigs, specialized laboratory and medical equipment such as magnetic resonance imaging and tomography systems, virtual and augmented reality devices such as VR/AR headsets and controllers, home automation equipment such as sensors and control systems, industrial and commercial Internet of Things (IoT) devices, satellite communication equipment and parabolic antennas, tracking devices such as GPS units and locators, 3D printing equipment and 3D scanners, small-scale wind energy systems, hydroponic cultivation systems and agricultural control systems, advanced scientific research equipment such as particle accelerators, thermal and geothermal energy generation systems, and alternative entertainment devices such as drones and flight simulators.

### Process for Treating WEEE

There are preliminary steps prior to carrying out the WEEE leaching process. In this regard, the raw material, namely WEEE, must first be obtained, which will subsequently be subjected to the leaching process.

Waste Electrical and Electronic Equipment (WEEE) is obtained from various sources, such as collection companies, recycling companies, collection points, and/or WEEE-generating entities (e.g., private telecommunications companies, the photovoltaic industry, public institutions, and the general public, among others). The raw material processed may include printed circuit boards (PCBs), photovoltaic cells, LED/LCD displays, electronic components in general, and may also include inorganic materials of mineral origin. The process applies to PCBs or photovoltaic cells contained within WEEE, that is, to the electrical and electronic components that contain metals not directly recoverable by simple mechanical separation, such as certain aluminum fractions present in the structural parts of specific WEEE. The raw material entering the process is considered to be substantially free of plastic casings and metallic parts that can be directly removed. Accordingly, WEEE fed into the process may either be pre-cleaned from such plastic components (when originating from recycling companies) or be disassembled to obtain the electrical and electronic components (when originating from general collection sources).

In a second stage, the raw material is subjected to a characterization to determine its origin (category and type of industrial activity).

The initial characterization of inorganic solid WEEE materials is carried out in order to determine the origin of the different residues according to their source of generation, category or type, manufacturing origin, technological generation, or mineral source (if applicable).

Depending on the origin, different types of WEEE are separated in order to avoid uncontrolled mixing of raw materials. Based on the origin of the WEEE, process parameters and operating condition ranges for the leaching solution are defined. By way of example, when the feed material corresponds to photovoltaic cells, the solids concentration in the process slurry may be controlled within a range of 5 to 35%, the particle size distribution of the raw material may reach an F80 between 5 and 25 mm, the leaching solution replacement cycles may be carried out up to the fourth day of the process, and the composition of the organic leaching solution may include sulfuric acid in a concentration ranging from 0.1 to 25 g/L, in order to direct lead to either the liquid or solid product, depending on market requirements.

By way of example, when the feed material corresponds to printed circuit boards (PCBs), the solids concentration in the process slurry may be controlled within a range of 1 to 20%, the raw material may have an F80 ranging from 3 to 15 mm, the leaching solution replacement cycles may be carried out starting from the second day of the process, and the organic leaching solution may include sulfuric acid at a concentration ranging from 0.1 to 25 g/L.

On the other hand, this stage is also used to support process-related decisions, such as separating different types of WEEE into batches according to their metal grade, classified as high grade (above 30% Cu), medium grade (between 20% and 30% Cu), and low grade (below 20% Cu), for the purpose of chemical characterization, mass balance calculations, and preventing uncontrolled mixing of raw materials.

The WEEE leaching process of the present invention comprises the following steps:
i. crushing the WEEE to obtain particles having an F80 between 3 and 25 mm.
   This size reduction stage is carried out using mechanical equipment in order to reduce and homogenize the particle size distribution to a range suitable for hydrometallurgical processing. Accordingly, the process may be performed using toothed roll crushers or jaw crushers (depending on the type of raw material), operating in closed circuit with a size classifier having a cut-off screen at 25 mm.
ii. loading the crushed WEEE up to a solids concentration of 35% into a reactor-type vessel, flooding with the BioReagent or leaching solution, agitating the mixture at a speed ranging from 250 to 800 rpm, homogenizing and maintaining a temperature between 35 and 85 °C, with forced aeration at a rate of 0.08 to 0.25 L air/min/L slurry, at atmospheric pressure (1 atm), for a period of 5 to 15 days.
   At this stage, the leaching of the metals of interest contained in the crushed materials is carried out. Selectively, copper is dissolved into the leaching solution. This agitated leaching process may be performed in batch or continuous mode.
iii. replacing the loaded leaching medium with fresh solution and incorporating fresh feed material into the process depending on the pH and/or Eh of the solution, the pH and/or Eh being monitored at intervals of 8 to 24 hours.

During the leaching period, the electrochemical conditions evolve (pH, Eh-SHE, electrical conductivity (EC), dissolved oxygen (DO), and temperature), and the solution progressively approaches saturation, thereby reducing its leaching capacity. Accordingly, the methodology requires replacement of the metal-laden leaching solution with fresh solution, as well as the introduction of fresh feed material into the process (i.e., a new charge of material under the same initial conditions). Thus, after the initial stage of the leaching process, it is necessary to discharge between 5% and 85% of the leaching solution and incorporate a substantially equivalent volume of fresh BioReagent solution.

In the leaching of Waste Electrical and Electronic Equipment (WEEE), the leaching solution is replaced after 2 and 4 days of leaching. Thereafter, at approximately day 5 and/or day 8 of the leaching process, replacement is evaluated and carried out only if required by the prevailing process conditions.

If the redox potential (Eh) is below 500 mV (vs. SHE), forced aeration is maintained. If the Eh is greater than or equal to 500 mV (vs. SHE), forced aeration is discontinued.

If the pH is greater than or equal to 3.5, solution replacement may be carried out together with the addition of sulfuric acid at the same initial concentration as that present in the BioReagent.

Additionally, at each solution replacement, fresh feed material may be introduced into the process without discharging the material initially charged, the introduced feed having a mass equivalent to the mass initially charged.

After completing the replacement, the leaching process is restarted by controlling the agitation, temperature, and aeration parameters, and by monitoring the electrochemical conditions at intervals of 8 to 24 hours.

Redox potential (Eh) range between 150-850 mV vs. SHE.

If the Eh is between 150-500 mV vs. SHE, forced aeration shall be maintained.

If the Eh is between 500-850 mV vs. SHE, forced aeration shall be stopped.

pH range between 0.2-4.5.

The pH condition is verified at intervals of 8-24 hours, with an acceptable operating range between 0.2-3.5.

If the pH is between 3.5 and 4.5, solution replacement is carried out on days 2 and 4 of leaching. Between days 5 and 10, an initial sulfuric acid dosage at the same concentration as that used in the initial solution is evaluated. If, after 24 hours, the pH does not decrease to the acceptable range, the solution is replaced with fresh BioReagent solution.

iv. the leaching process is terminated by stopping agitation and forced aeration, thereby allowing the solids contained in the slurry to settle for a period of 40 to 60 minutes.

v. separating and discharging the copper-enriched solution (PLS) from the slurry obtained in step (iv).

The copper-enriched solution is pumped to at least one storage unit, wherein homogenization is achieved by mechanical agitation and/or by recirculation of the PLS within said storage unit so as to prevent the formation of concentration gradients.

The copper-enriched solution (PLS) is continuously or periodically monitored with respect to its electrochemical parameters (pH/Eh/EC/DO), and samples are withdrawn for analytical characterization.

vi. discharging the pulp concentrated in solids obtained in step (v), which is subjected to solid-liquid separation by filtration, followed by drying at a temperature between 60 and 90 °C for 12 to 30 hours.

The filtration stage may be carried out by pressure filtration, vacuum filtration, membrane filtration, filter press filtration, tangential filtration, centrifugation, or gravity filtration.

vii. sieving the filtered and dried solid material obtained in step (vi) using a screen or vibrating equipment fitted with a mesh having an aperture size between 250-1000 µm, thereby obtaining a fine product and a coarse spent leach residue.

The material passing through the mesh aperture between 250-1000 µm is defined as the fine product, and the retained or oversize material is defined as the coarse spent leach residue. Mass measurements are performed on both solid products in order to assess deviations, and representative samples are collected. The coarse spent leach residue is directed to final disposal or recycled.

### Analysis of metal concentration in the PLS and solid product

Samples obtained from the fine product and the PLS are subjected to chemical analysis in order to quantitatively determine the concentration of metals of interest and potential contaminants. Accordingly, the fine product sample is analyzed for grades of Au, Ag, Pd, Pt, Pb, As, Cd, and other elements relevant for commercial valuation or identification of penalty elements. Similarly, the PLS is analyzed to determine the concentration of Cu, Fe, Pb, As, Au, Ag, and other elements of interest for commercial valuation or regulatory compliance purposes.

The present invention demonstrates the technical feasibility of the leaching of shredded WEEE using a BioReagent formulated from residues originating from the wine-making, forestry and/or agricultural industries, in accordance with the proposed classifications. In addition, mixtures of residues from different industries (e.g., winemaking-forestry or fruit-processing industries) may be suitably combined, thereby enabling adjustment and proportioning of the organic components, which reduces the risk associated with raw material supply for the preparation of the BioReagent.

The advantages of the present invention are set forth below:
1. Circular process based on the utilization of organic residues from the wine-making, forestry and/or agricultural industries.
2. Circular process for the valorization of waste electrical and electronic equipment (WEEE).
3. A leaching process that is technically feasible using mixtures of different organic residues.
4. Utilization of naturally derived or organic-origin chemical agents recovered from industrial waste residues.
5. Process involving the application of saline-chloride media, such as seawater and discarded salts from lithium production or desalination processes.
6. A hydrometallurgical leaching process carried out under atmospheric pressure (1 atm) conditions.
7. Leaching process carried out at intermediate temperature conditions.
8. Agitated leaching process employing feed material comminuted to millimeter-scale particle sizes, as opposed to micron-scale powders.
9. Leaching process over short time periods (days).
10. Production of two types of products: a copper-rich PLS (with low levels of contaminants or heavy metals that may affect subsequent refining stages); and a dry, fine solid product concentrated in gold, silver, palladium, platinum and/or indium (if applicable).
11. Leaching process conducted with a low concentration of strong oxidizing agents.
12. Environmentally friendly leaching process, as it does not use, for example, hydrochloric acid, nitric acid, hydrofluoric acid, or hydrocyanic acid; the process is free of cyanide, urea, nitrates, amines, amides, esters, and benzene.
13. Leaching process feasible to be carried out using mixtures of feed material to be processed.
14. Low energy consumption compared to pyrometallurgical processes or hydrometallurgical processes conducted in autoclaves at high temperatures and pressures.

### EXAMPLES OF APPLICATION

The organic BioReagent or leaching solution, and its application in the leaching of WEEE, as described in the present invention, were developed through various application examples, which are described below:

### EXAMPLE 1

Process for obtaining the organic BioReagent; the details of certain specific examples for obtaining the reagent of the present invention are set forth below, without limiting the invention to the examples described herein.

The following Table 1 summarizes the different groups of "fresh" organic residues processed for organic waste recovery.

| Table 1 | |
|---|---|
| Organic species (biomass) | Organic species code |
| Eucalyptus Nitens | D1 |
| Eucalyptus Globulus | |
| Eucalyptus Gloni | |
| Pinus Radiata (Insigne pine) | |
| Syrah | D2 |
| Non-traditional blend | |
| Cabernet Sauvignon | |
| Merlot | |
| Carmenere | |
| Mixtures between D1 and D2 | D3 |

Once the organic biomass was obtained, a characterization of its waste source was carried out, including identification of the type of industrial activity, geographical region, and species or organic strain. The characterization of the organic solids was performed in order to determine the origin of the different residues as a function of their age, species or organic strain, and geographical location.

Subsequently, a physical characterization of the obtained organic residue was carried out. Initial moisture content was determined by controlled drying in a forced-convection oven at 75 °C for 24 hours.

The moisture content may be considered an initial indicator of the preservation state or freshness of the organic material. Organic materials exhibiting higher moisture content (not altered by external factors) allow for adequate recovery of the acidic and/or phenolic compounds contained therein for incorporation into the leaching solution. In other words, if the moisture content is low, the resulting recovered solution tends to exhibit reduced strength. However, it should be noted that winemaking-derived organic materials may exhibit a degree of fermentation due to bacterial activity, resulting in the presence of different organic compounds such as lactic acid (formed from malic acid) or acetic acid.

Table 2: Moisture content of the organic groups in solid form used in the tests for the preparation of the BioReagent.

| Table 2: Moisture Content of Organic Groups in Solid Form | |
|---|---|
| Group | Moisture (%) |
| D1 | 35.7 |
| D2 | 57.3 |
| D3 **approx. 50%* | 46.5 |

Additionally, a chemical characterization of the obtained organic residue (organic acids, phenolic compounds, and total acidity) was carried out, and the corresponding data are presented in Tables 3, 4, and 5. The chemical characterization was performed using various analytical methodologies, including molecular absorption spectrophotometry, high-performance liquid chromatography (HPLC), and enzymatic assay kits, for the determination of polyphenols and organic acids including acetic, malic, lactic, tartaric, and citric acids. In addition, total acidity was determined by potentiometry, and electrochemical variables including pH, Eh (oxidation-reduction potential), and EC (electrical conductivity) were monitored.

**Table 3. Characterization of natural acids and polyphenols from the different organic groups (liquid format).**

| Table 3 | | | | | |
|---|---|---|---|---|---|
| Group | Total Acidity (mg/kg) | Tartaric Acid (mg/kg) | Malic Acid (mg/kg) | Acetic Acid (mg/kg) | Polyphenols (mg/kg) |
| D1 | 933 | - | - | - | 30068 |
| D2 | 11800 | 16480 | 600 | 640 | - |
| D3 | 7136 | 30000 | 40 | 2140 | 24612 |

**Table 4. Citric and malic acid concentrations in the D2 organic group, determined in solid samples.**

| | Table 4 | | | | |
|---|---|---|---|---|---|
| | D2 | Citric Acid (mg/kg) | | Malic Acid (mg/kg) | |
| | Sample 1 | | 47 | | 485 |
| | Sample 2 | | 45 | | 458 |

The process temperature varied depending on the specific application required for the recovery of organic agents. For coniferous- or Myrtaceae-type organic materials (D1), the temperature ranged from 30-60 °C; for Vitaceae- and Rosaceae-type organic materials (D2), from 50-95 °C; and for mixtures thereof (D3), from 40-75 °C, as a function of processing time and organic concentration. Higher organic concentrations and longer processing times allowed the treatment to be conducted at lower temperatures.

The temperature-controlled recovery process applied to D1- and D2-type residues, as well as D3 mixtures, was carried out for 6 hours at 60 °C. Maintaining this temperature enhances reaction kinetics and increases the recovery of organic agents by up to 61%, after which a stable chemical and electrochemical state is achieved.

In addition, the electrochemical parameters are established as preliminary and overall indicators for assessing the suitability of the enriched organic solutions, since specific analytical determinations of individual analytes are required using the aforementioned techniques, which enable evaluation of process sensitivity and variability.

Additionally, it has been determined that the curing period in the D2 organic group constitutes a variable affecting the condition of the BioReagent, resulting in increases of up to twenty-fold in acetic acid concentration, a 111% increase in total acidity, and a 27% decrease in tartaric acid concentration. Furthermore, the use of fresh organic material results in conditions favorable for hydrometallurgical metal recovery.

Finally, higher organic concentrations in the preparation result in increased concentrations of polyphenols, total acidity, and organic acids. However, at lower organic concentrations, conditions potentially suitable for hydrometallurgical metal recovery are achieved, thereby simplifying the solid-liquid separation and drying stages.

The following section presents selected results from the preparation of the BioReagent.

### Organic residue treatment stage

Several tests were conducted under the following operating conditions for the D1 sample analyzed: a processing time of 6 hours at 60 °C. The following tests were performed: Test E6 (D1 Nitens, 300 g/L); Test E16 (D1 Gloni, 300 g/L); Test E17 (D1 Globulus, 50 g/L); Test E20 (D1 Globulus, 300 g/L); and Test E36 (D1 Gloni, 300 g/L).

Table 5 presents the polyphenol concentration and equivalent total acidity obtained from the processing of D1 group organic residues.

| Table 5 | | | | | | | | | |
|---|---|---|---|---|---|---|---|---|---|
| Test | Total Acidity (g/L) | | Polyphenol concentration (g/L) | | pH | Electrical Conductivity (µS/cm) | | Eh (mV vs. SHE) | |
| E6 | | 2.11 | | 9.1 | 3.6 | | 2524 | | - |
| E16 | | 1.44 | | 7.9 | 4.2 | | 2879 | | - |
| E17 | | 0.35 | | 2.3 | 4 | | 548 | | - |
| E20 | | 1.6 | | 10.6 | 4 | | 2418 | | 385.9 |
| E36 | | 1.81 | | 7.8 | 3.8 | | 2213 | | 447.5 |

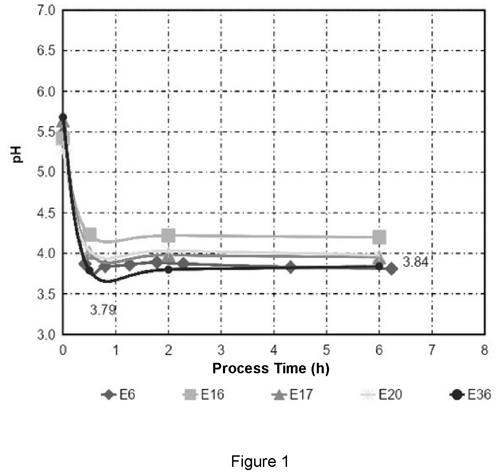

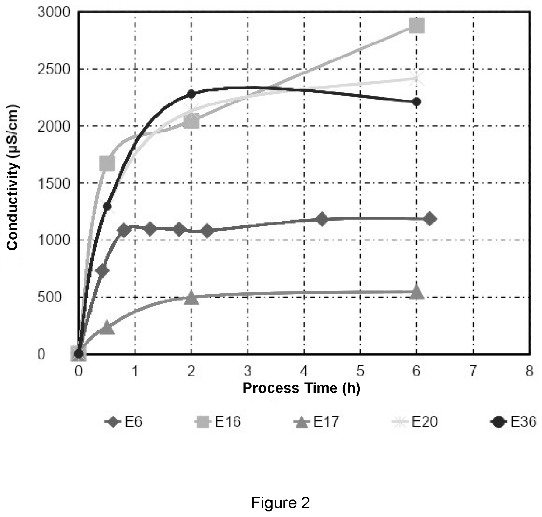

The results are presented in Figures 1 and 2. Figure 1 illustrates the evolution of pH in Tests E6, E16, E17, E20, and E36, while Figure 2 illustrates the evolution of electrical conductivity during the D1 organic residue processing stage.

Subsequently, catalysts were added to the obtained solutions. The catalysts included sulfuric acid, chloride ions, iron, and/or microorganisms. Table 6 presents the concentrations (g/L) employed for the D1 sample.

Table 6 shows a summary of the conditions and catalysts used in the preparation of the BioReagent from D1-type organic residues.

| Table 6 | | | | | | | | | | |
|---|---|---|---|---|---|---|---|---|---|---|
| Solution | Total Acidity (g/L) | | Polyphenol concentration (g/L) | | Catalysts | | | | | |
| | | | | | Sulfuric Acid (g/L) | | Chloride (g/L) | | Iron (g/L) | |
| E8 | | 2.11 | | 9.1 | | 5.0 | | 19 | | 5.0 |
| E16 | | 1.44 | | 7.9 | | 5.0 | | 19 | | 5.0 |
| E17 | | 0.35 | | 2.3 | | 5.0 | | 19 | | 5.0 |
| E20 | | 1.6 | | 10.6 | | 5.0 | | 19 | | 5.0 |
| E36 | | 1.81 | | 7.8 | | 5.0 | | 19 | | 5.0 |

### EXAMPLE 2

Several tests were conducted under the following operating conditions for the analyzed D2 sample: a processing time of 6 hours at 60 °C. The tests performed were: Test E43 (D2 Syrah, 300 g/L); Test E44 (D2 Syrah fermented for 15 days, 300 g/L); Test E45 (D2 organic mixture derived from Tests E43 and E44, 150 g/L each); and Test E29 (D2 Syrah, 5 g/L).

Table 7 summarizes the concentrations of total acidity, tartaric acid, malic acid, and acetic acid obtained during the processing of D2-type organic residues.

| Table 7 | | | | | |
|---|---|---|---|---|---|
| Test | Total Acidity (g/L) | Tartaric Acid (g/L) | | Malic Acid (g/L) | Acetic Acid (g/L) |
| E29 | 0.59 | | 1.623 | 0.074 | 0.032 |
| E43 | 2.21 | | 5.9 | 0.147 | 0.107 |
| E44 | 4.66 | | 4.3 | 0.062 | 2.267 |
| E45 | 3.38 | | 5.05 | 0.118 | 1.357 |

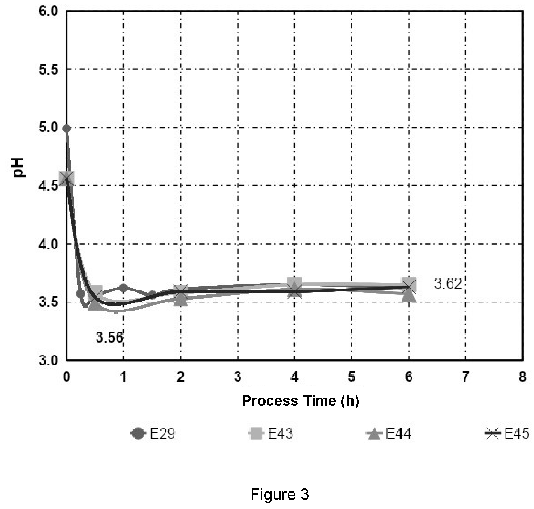

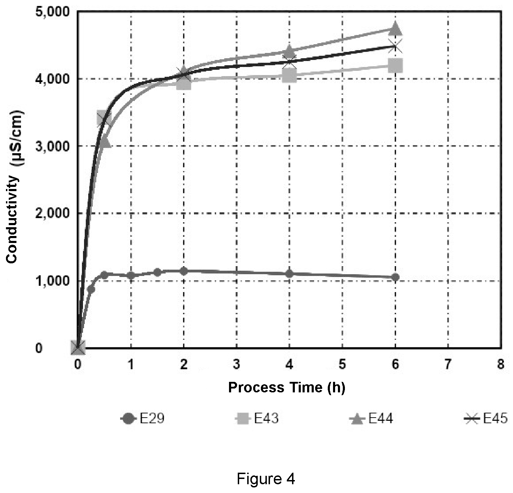

The results are presented in Figures 3 and 4. Figure 3 illustrates the evolution of pH in Tests E29, E43, E44, and E45, while Figure 4 illustrates the evolution of electrical conductivity during the D2 organic residue processing stage.

Subsequently, catalysts were added to the obtained solutions. The catalysts may comprise sulfuric acid, chloride ions, iron, and/or microorganisms. Table 8 presents the concentrations (g/L) used for the D2 sample.

Table 8 shows a summary of the operating conditions and catalysts employed in the preparation of the BioReagent from D2 organic residues.

| Table 8 | | | | | | | |
|---|---|---|---|---|---|---|---|
| Solution | Total Acidity (g/L) | Tartaric Acid (g/L) | Malic Acid (g/L) | Acetic Acid (g/L) | Catalysts | | |
| | | | | | Sulfuric Acid (g/L) | Chloride (g/L) | Iron (g/L) |
| E29 | 0.59 | 1.623 | 0.074 | 0.032 | 5.0 | 19 | 5.0 |
| E43 | 2.21 | 5.9 | 0.147 | 0.107 | 5.0 | 19 | 5.0 |
| E44 | 4.66 | 4.3 | 0.062 | 2.267 | 5.0 | 19 | 5.0 |
| E45 | 3.38 | 5.05 | 0.118 | 1.357 | 5.0 | 19 | 5.0 |

### EXAMPLE 3. Treatment of WEEE

For the examples, leaching solutions or BioReagent compositions comprising the components indicated in Table 9 were used.

| Table 9 | | | | | | | | |
|---|---|---|---|---|---|---|---|---|
| Base Solution | Residue Type | Total Acidity (g/L) | Tartaric Acid (g/L) | Malic Acid (g/L) | Acetic Acid (g/L) | Catalysts | | |
| | | | | | | Sulfuric Acid (g/L) | Chloride (g/L) | Iron (g/L) |
| B1.0 | D2 | 0.59 | 1.623 | 0.074 | 0.032 | 5.0 | 19 | 5.0 |

| Base Solution | Residue Type | Total Acidity (g/L) | Tartaric Acid (g/L) | Malic Acid (g/L) | Acetic Acid (g/L) | Catalysts | | |
|---|---|---|---|---|---|---|---|---|
| | | | | | | Sulfuric Acid (g/L) | Bacterial inoculum at a concentration of 1 × 10⁶ cells/mL | Iron (g/L) |
| B2.0 | D2 | 0.59 | 1.623 | 0.074 | 0.032 | 3.0 | 100 | 5.0 |

| Base Solution | Residue Type | Total Acidity (g/L) | Polyphenol (g/L) | Catalysts | | |
|---|---|---|---|---|---|---|
| | | | | Sulfuric Acid (g/L) | Chloride (g/L) | Iron (g/L) |
| B3.0 | D1 | 2.11 | 9.1 | 5.0 | 11 | 5.0 |

The WEEE groups to be treated were divided into five inorganic categories in order to evaluate the sensitivity of the different organic agents as a function of the treatment matrix. The inorganic material consisted of printed circuit boards (PCBs) from electrical and electronic equipment, which were categorized as set forth in Table 10.

| | Table 10 | |
|---|---|---|
| | General description of feedstock for hydrometallurgical leaching processes | |
| RAW MATERIAL CODE | | GENERAL DESCRIPTION |
| | RA1 | Corresponds to computer PCBs. |
| | | Physical condition: fine. |
| | RA2 | Corresponds to cell phone PCBs |
| | | Physical condition: fine. |
| | RA3 | Corresponds to a mixed group comprising all WEEE categories in general. |
| | | Physical condition: fine. |
| | RA4 | Corresponds to a mixed group comprising all WEEE categories in general. |
| | | Physical condition: crushed or coarse. |
| | RA5 | Corresponds to a group comprising photovoltaic cells. |
| | | Physical condition: crushed or coarse. |

Once the WEEE feed material was received, a physical characterization was carried out, including specific gravity, mass, and bulk density measurements. At this stage, mass controls were performed before and after crushing in order to prevent deviations in key performance indicators (KPIs) and to obtain relevant information for subsequent metallurgical mass balances. Specific gravity (where applicable) and bulk density were also determined, and visual inspection by means of optical microscopy was conducted to evaluate the distribution of metals of interest. In addition, chemical analyses were performed to determine head grades (feed grades to the process), corresponding to the concentrations of metals of interest and/or contaminants, using Atomic Absorption Spectroscopy (AAS), Fire Assay (FA), Inductively Coupled Plasma Optical Emission Spectroscopy (ICP-OES), and/or X-ray Fluorescence (XRF), as appropriate depending on the feed material.

### Bulk Density of Inorganic Treatment Groups

Table 11 presents a summary of the bulk density values for each of the WEEE groups processed in the different leaching tests. An average bulk density of 1.005 t/m³ was established, primarily influenced by the plastic material content in the electronic boards or PCBs.

| Table 11 | |
|---|---|
| Raw Material Code | Bulk Density (t/m³) |
| RA1 | 0.987 |
| RA2 | 1.021 |
| RA3 | 1.008 |
| RA4 | 0.786 |
| RA5 | 0.220 |

### Particle Size Analysis of Inorganic Treatment Groups

Table 12 shows the particle size distribution of the inorganic groups used in the technical validation processes of the BioReagent.

| Table 12 | | |
|---|---|---|
| Raw Material Code | F(80) (mm) | F(50) (mm) |
| RA1 | 3.14 | 4.04 |
| RA2 | 3.29 | 4.19 |
| RA3 | 3.28 | 4.18 |
| RA4 | 8.110 | 5.780 |
| RA5 | 9.700 | 7.200 |

Based on the characterization of the WEEE, process parameters and ranges of operating conditions for the leaching solution were defined. Thus, bulk density, together with the previously defined solids concentration and the estimated pulp density, allowed calculation of the maximum volume of leaching solution to be loaded into the equipment in order to maintain an effective filling factor of 85%.

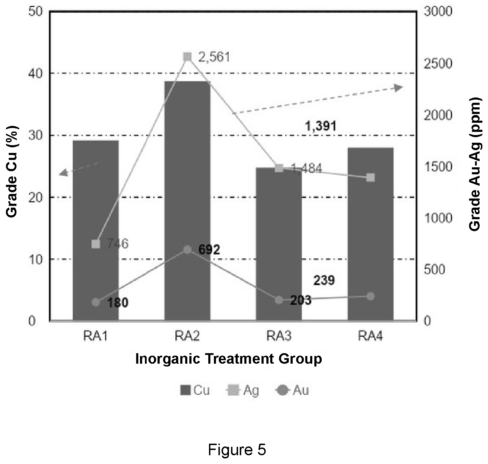

On the other hand, feed material of different origins and physicochemical characteristics intended for the leaching process was controlled and incorporated into the process in selected proportions of high-, medium-, and low-grade material in order to maintain metal concentrations within a suitable operating range for the process. Thus, based on the characterization of the PCB material, the feed introduced into the process was controlled to achieve average grades within the ranges of 20-35% for Cu, 150-850 ppm for Au, and 500-2000 ppm for Ag, as shown in Figure 5.

### Chemical Analysis of Metals and Other Elements

The chemical characterization based on ICP mass spectrometry (ICP-MS) analyses of the metals present in the three WEEE groups is presented in Table 13.

Table 13 shows a summary of the ICP analysis of the metals present in the inorganic treatment groups.

| Table 13 | | | | | | | | | |
|---|---|---|---|---|---|---|---|---|---|
| WEEE Type | Metals | | | | | | | | |
| | Ag (%) ICP | Ba (%) ICP | Ca (%) ICP | Cu (%) ICP | Fe (%) ICP | Mg (%) ICP | Na (%) ICP | Sn (%) ICP | Zn (%) ICP |
| RA1 | 0.07 | 0.91 | 4.78 | 23.94 | 0.88 | 0.17 | 0.10 | 5.64 | 0.91 |
| RA2 | 0.24 | 1.12 | 3.1 | 37.71 | 1.77 | 0.15 | 0.07 | 2.56 | 0.24 |
| RA3 | 0.15 | 0.82 | 4.97 | 23.25 | 0.70 | 0.12 | 0.09 | 5.46 | 0.59 |

Table 14 presents the results of the Cu, Au, and Ag grades determined by ICP analysis, Atomic Absorption Spectroscopy (AAS), volumetric analysis (vol.), and Fire Assay (FA/AAS).

The characterization of the inorganic solids (WEEE) was carried out in order to quantify the metals present in the different assigned categories. A first stage consisted of determining origin, technology, and production context. A second stage involved chemical analyses to determine the concentration of the metals present, using Atomic Absorption Spectroscopy (AAS), complemented by X-ray Fluorescence (XRF), X-ray Diffraction (XRD), Inductively Coupled Plasma Mass Spectrometry (ICP), Fire Assay (FA), and QEMSCAN (Quantitative Evaluation of Minerals by Scanning Electron Microscopy).

| Table 14 | | | | | | | |
|---|---|---|---|---|---|---|---|
| WEEE Type | Cu | | | Ag | | Au | |
| | ICP (%) | %AAS | % Vol, | ICP (mg/kg) | AAS (mg/kg) | FA/AAS (mg/kg) | AAS (mg/kg) |
| RA1 | 23.9 | 28.2 | 29.2 | 658 | 746 | 84 | 180 |
| RA2 | 37.7 | 37.8 | 38.7 | 2409 | 2561 | 318 | 692 |
| RA3 | 23.3 | 24.3 | 24.8 | 1450 | 1484 | 215 | 203 |
| RA4 | - | 31.0 | 28.0 | - | 1391 | - | 239 |
| RA5 | - | 5.43 | - | 442 | - | - | - |

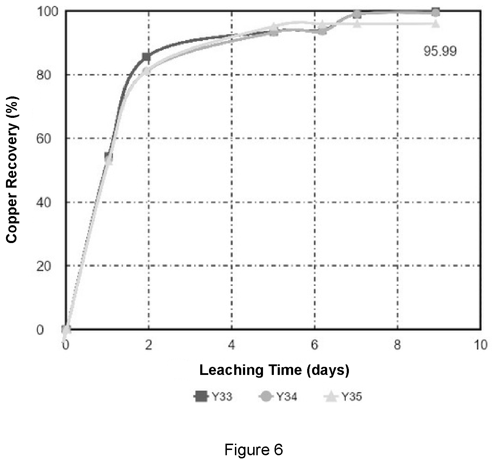

Tests were conducted to determine copper recovery for sample RA4. The tests were carried out at a process temperature of 60 °C over a leaching period of 9 days, using a leaching solution prepared from D1-type residue, to which catalysts were added as indicated in Table 15. Figure 6 shows the copper recovery kinetics for sample RA4 obtained in Tests Y33, Y34 (duplicates), and Y35.

Additionally, Test Y35 was conducted using a mixture in the preparation of the D1- and D2-type organic residues at a ratio of 0.25:0.75, respectively.

Two controlled solution replacement cycles were carried out on days 2 and 4 of the process. Additionally, on day 2, a fresh PCB sample was incorporated (without removing solids from the system) at the same mass initially introduced at the start of the process (initial pulp solids content: 3%). Tests Y33 and Y34 achieved copper recoveries above 95%, reflecting an improvement of 3 percentage points compared to Test Y35 conducted with the organic mixture. This is attributed to the fact that, in combination with the solution replacement cycles, the mixed-type BioReagent requires a longer conditioning period in contact with the processed PCB. Nevertheless, the results are highly favorable, and it is technically feasible to manage the leaching solution derived from residues of different origins.

| Table 15 | | | | | | |
|---|---|---|---|---|---|---|
| Base Solution | Residue Type | Total Acidity (g/L) | Polyphenol (g/L) | Catalysts | | |
| | | | | Sulfuric Acid (g/L) | Chloride (g/L) | Iron (g/L) |
| B3.0 | D1 | 2.11 | 9.1 | 5.0 | 11 | 5.0 |

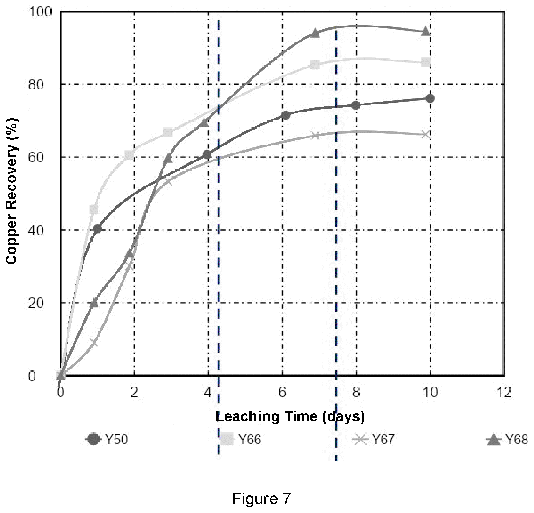

Figure 7 shows the copper recovery kinetics during leaching of RA4 inorganic material, comparing BioReagents B1.0 and B2.0 as indicated in Table 16 in the processing of PCB-type WEEE. The process was conducted at 60 °C for Tests Y66, Y67, and Y68. Test Y50 was carried out at 40 °C with the application of a bacterial inoculum. The leaching solution was prepared from D2-type Syrah organic residue. Test Y50 (using BioReagent B2.0) was performed under continuous operation without solution replacement or incorporation of fresh sample into the process, achieving a copper recovery of 76% after 10 days of leaching. In contrast, for the tests using BioReagent B1.0, operational adjustments were implemented. Test Y66 involved a single solution replacement control cycle on day 2 of leaching, without incorporation of fresh sample. Test Y67 involved a single solution replacement control cycle on day 2 of leaching, with incorporation of fresh sample at the same mass as initially introduced. Finally, Test Y68 involved two control cycles on days 2 and 4 of leaching, incorporating fresh sample only during the first cycle (day 2). The most favorable results were obtained in Test Y68, achieving a copper recovery of 95% after 10 days of leaching.

| Table 16 | | | | | | | | |
|---|---|---|---|---|---|---|---|---|
| Base Solution | Residue Type | Total Acidity (g/L) | Tartaric Acid (g/L) | Malic Acid (g/L) | Acetic Acid (g/L) | Catalysts | | |
| | | | | | | Sulfuric Acid (g/L) | Chloride (g/L) | Iron (g/L) |
| B1.0 | D2 | 0.59 | 1.623 | 0.074 | 0.032 | 5.0 | 19 | 5.0 |

| Base Solution | Residue Type | Total Acidity (g/L) | Tartaric Acid (g/L) | Malic Acid (g/L) | Acetic Acid (g/L) | Catalysts | | |
|---|---|---|---|---|---|---|---|---|
| | | | | | | Sulfuric Acid (g/L) | Bacterial inoculum at a concentration of 1 × 10⁶ cells/mL | Iron (g/L) |
| B2.0 | D2 | 0.59 | 1.623 | 0.074 | 0.032 | 3.0 | 100 | 5.0 |

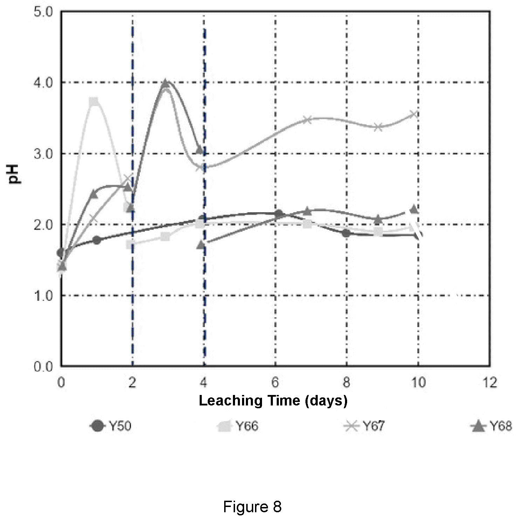

Figure 8 shows the evolution of pH during leaching of the RA4 inorganic material, evaluating BioReagents B1.0 and B2.0 in the processing of PCB-type WEEE. The process was conducted at 60 °C for Tests Y66, Y67, and Y68, and at 40 °C for Test Y50 with the application of a bacterial inoculum. The leaching solution was prepared from D2-type Syrah organic residue.

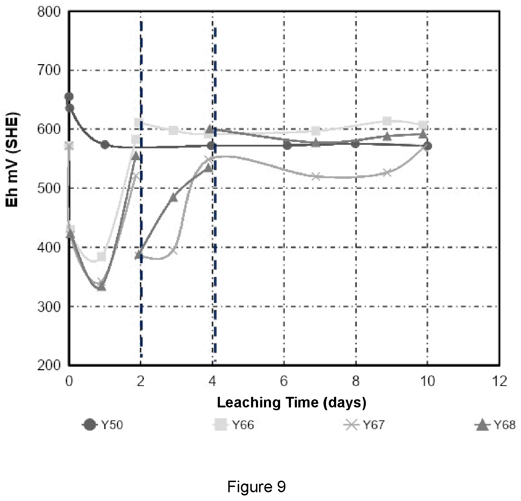

Figure 9 shows the redox potential (Eh) evolution during leaching of RA4 inorganic material, comparing BioReagents B1.0 and B2.0 in the processing of PCB-type WEEE. The process was conducted at 60 °C for Tests Y66, Y67, and Y68, and at 40 °C for Test Y50 with the application of a bacterial inoculum. The leaching solution was prepared from D2-type Syrah organic residue.

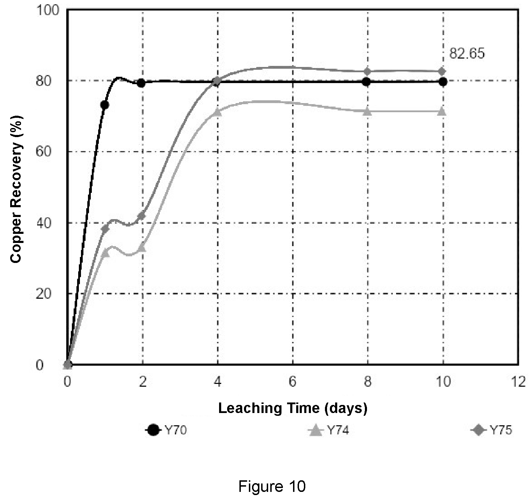

Figure 10 shows the copper recovery kinetics during leaching of RA5 inorganic material using BioReagent B1.0 in the processing of photovoltaic cell WEEE. The process was conducted at 60 °C. The leaching solution was prepared from D2-type Syrah organic residue, as indicated in Table 17. Tests Y70 and Y75 (duplicate runs) included solution replacement cycles on days 2 and 4 of the process, with incorporation of fresh sample on day 2 during the first replacement cycle, the fresh sample being introduced at the same mass as initially charged. Test Y74 included a single solution replacement cycle on day 2 of the process.

| Table 17 | | | | | | | | |
|---|---|---|---|---|---|---|---|---|
| Base Solution | Residue Type | Total Acidity (g/L) | Tartaric Acid (g/L) | Malic Acid (g/L) | Acetic Acid (g/L) | Catalysts | | |
| | | | | | | Sulfuric Acid (g/L) | Chloride (g/L) | Iron (g/L) |
| B1.0 | D2 | 0.59 | 1.623 | 0.074 | 0.032 | 5.0 | 19 | 5.0 |

The technical results for metal recovery from the RA5 group are highly favorable, with an average copper recovery above 75% (see Figure 10). In addition, silver recovery exceeded 65%, reaching silver grades of 1100 ppm in the solid product after a processing time of 10 days. In RA5 processing, a certain degree of uncertainty exists due to the heterogeneity of the photovoltaic cell samples; however, there is also significant potential for process optimization through the establishment of an appropriate electrochemical operating range. Furthermore, depending on the applied process conditions (particularly the leaching solution concentration), heavy metals such as Pb may increase in concentration in solution by up to tenfold. Finally, the highest copper recovery was obtained in Test Y75, reaching 82% after 10 days under medium-temperature conditions (60 °C) and low catalyst concentrations.

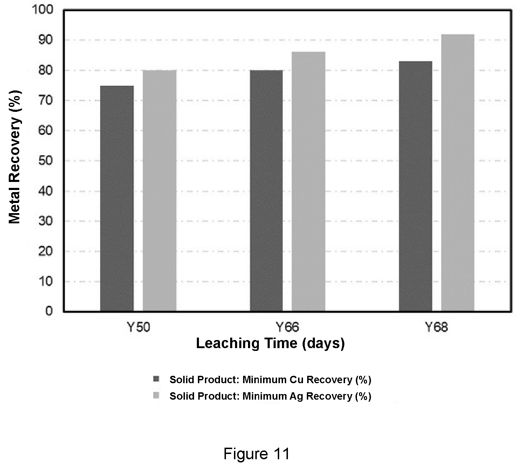

Figure 11 shows the gold and silver recovery results for Tests Y50, Y66, and Y68, according to the methodology previously described. The filtered solids from the processes were dried and sieved to a particle size below 600 µm. Test Y68 achieved gold and silver recoveries of 83% and 92%, respectively (dark gray bars = gold; light gray bars = silver). In contrast, Test Y50 achieved gold and silver recoveries of 75% and 80%, respectively.

The most suitable conditions for the application of the BioReagent in the leaching process of shredded WEEE achieved copper recoveries above 95% within an electrochemical window for selective copper dissolution, defined by a pH range of 1.30-3.99 and a potential range of 350-600 mV vs. SHE. In addition, silver and gold were recovered in the solid phase within the fine composite concentrate fraction below 600 µm, with recoveries exceeding 85%.

The PLS obtained from the leaching of shredded WEEE exhibited a composition comparable to conventional hydrometallurgical mining products. Copper concentrations ranged from 4 to 20 g/L, and iron from 1 to 3 g/L. No significant levels of contaminants such as arsenic, cadmium, or antimony were detected, while lead and aluminum levels were below 0.01 g/L and 0.08 g/L, respectively. Trace-level concentrations of other elements were identified, rendering the product suitable for subsequent hydrometallurgical processes (SX-EW), as shown in Table 18. Control of potential contaminants that may be present in the fresh feed material (e.g., photovoltaic cell-type WEEE) is achieved through the formulation of the BioReagent or leaching solution, which enables selective partitioning of contaminants such as Pb into either the liquid PLS phase or the solid composite fraction, as required.

Table 18. Characterization of Contaminant Elements Present in PLS-Type Products.

| Table 18 | | |
|---|---|---|
| Element | Concentration | Unit |
| Antimony | < 0.1 | mg Sb/L |
| Arsenic | < 2 | mg As/L |
| Beryllium | < 0.5 | mg Be/L |
| Boron | < 1 | mg B/L |
| Cadmium | < 0.2 | mg Cd/L |
| Vanadium | < 0.2 | mg V/L |
| Gallium | < 1 | mg Ga/L |
| Lanthanum | < 1 | mg La/L |
| Tellurium | < 1 | mg Te/L |
| Thallium | < 1 | mg Tl/L |
| Chromium | 0.403 | mg Cr/L |
| Bismuth | 0.465 | mg Bi/L |
| Barium | 0.501 | mg Ba/L |
| Lead | 11.23 | mg Pb/L |

The solid composite product containing Au and Ag obtained from the leaching of shredded WEEE exhibits a particle size below 600 µm, with no intermediate coarse fraction (above Tyler 30 and below 12 mm) suitable or enriched in metals after treatment. Its chemical composition shows more than a sixfold increase in Au grade and more than a fourfold increase in Ag grade compared to the fresh, unprocessed WEEE sample. It is possible to obtain Au and Ag grades ranging from 660 to 1600 ppm and from 2700 to 5600 ppm, respectively, depending on the quality of the fresh feed material introduced into the process.

The importance of implementing operational control cycles (including solution replacement and incorporation of fresh material) lies in the following:
First, it enables optimization of the BioReagent application by maintaining a selective copper dissolution window while retaining noble metals in the solid phase, as a function of the electrochemical conditions of the medium and the gradual or controlled increase in solids content in the pulp;
Second, it optimizes the quantity of products generated, both in the PLS and in the gold, silver, and palladium concentrates;
Third, it reduces CAPEX (Capital Expenditures) by reducing the number of equipment units required for pilot- or industrial-scale operation, since a greater amount of fresh feed material can be processed within the same time period; and
Fourth, it reduces OPEX (Operating Expenditures) by reducing the consumption of reagents and process inputs required for the production of PLS solutions and solid composite or concentrate products.

## Claims

1. A BioReagent or leaching solution, **CHARACTERIZED IN THAT** it comprises:
i. at least one of the following acids derived from organic residues obtained from waste generated by the wine-making, forestry and/or agricultural industries, such as: tartaric acid at a concentration ranging from 0 to 15 g/L, malic acid at a concentration ranging from 0 to 5.5 g/L, acetic acid at a concentration ranging from 0 to 6.5 g/L, citric acid at a concentration ranging from 0 to 5.0 g/L, and polyphenols at a concentration ranging from 0 to 20 g/L;
ii. a continental aqueous medium or a saline medium, wherein the aqueous medium may be continental water, also referred to as aquifer water or water from natural watercourses, distilled water, non-desalinated seawater, or saline discard streams derived from lithium production processes or from seawater desalination processes. The latter may reach chloride concentrations ranging between 45 and 85 g/L, originating from sodium salts;
iii. ferrous ions at a concentration ranging from 0.1 to 10 g/L;
iv. sulfuric acid at a concentration ranging from 0.1 to 25 g/L;
v. cupric ions at a concentration ranging from 0 to 10 g/L;
vi. an inoculum of mesophilic or thermophilic microorganisms (bacteria or archaea) at a concentration ranging from 0 to 150 × 10⁶ cells/mL, wherein the sulfur-oxidizing and/or iron-oxidizing bioleaching microorganisms are selected from Ferroplasma acidiphilum, Leptospirillum ferrooxidans, Acidianus infernus, Acidithiobacillus ferrooxidans, and Acidithiobacillus thiooxidans.

2. The BioReagent or leaching solution according to claim 1, **CHARACTERIZED IN THAT** in (i) the residues or waste generated by wine-making, forestry and/or agricultural industries are selected from coniferous species, myrtaceous species, Eucalyptus, pine, Vitaceae, grape pomace, Rosaceae, apple peels, peach residues, and fruit waste.

3. The BioReagent or leaching solution according to claim 1, **CHARACTERIZED IN THAT** in (ii) the discard salts may include other sources of chloride ions such as salts generated in lithium production processes, including bischofite, halite in solid form, and halite in liquid form.

4. A method for obtaining the BioReagent or leaching solution according to claim 1, **CHARACTERIZED IN THAT** it comprises:
(a) obtaining the organic residue and reducing its particle size to less than 6 inches (15.24 cm);
(b) loading the size-reduced organic residue from step (a) at a concentration ranging from 0.1 to 300 g/L into a closed temperature-controlled bath at atmospheric pressure (1 atm) containing an aqueous medium at a temperature ranging from 20 to 95 °C, homogenizing and aerating the mixture for a period ranging from 0.5 to 6 hours to produce an enriched organic solution;
(c) stopping step (b) by allowing the temperature to decrease, thereby obtaining a slurry comprising a liquid phase and organic residue;
(d) discharging the slurry obtained in step (c) and performing a solid-liquid separation once the medium has cooled to below 50 °C, by filtration until drainage of the solution ceases, thereby obtaining the loaded solution to be used for preparing the BioReagent, while the solid fraction is directed to composting;
(e) incorporating into the enriched solution from step (d) chemical agents functioning as catalysts, selected from:
- chloride ions derived from pure salts, such as halite, or from discard salts, at a concentration ranging from 0 to 90 g/L;
- ferrous ions at a concentration ranging from 0.1 to 10 g/L;
- sulfuric acid at a concentration ranging from 0.1 to 25 g/L;
- cupric ions at a concentration ranging from 0 to 10 g/L; and
- an inoculum of sulfur-oxidizing and/or iron-oxidizing microorganisms at a concentration ranging from 0 to 150 × 10⁶ cells/mL;
(f) homogenizing the solution from step (e) by mechanical agitation in a vertical vessel and/or by vibration until a homogeneous mixture is achieved, thereby obtaining the BioReagent or leaching solution.

5. The method for obtaining the BioReagent or leaching solution according to claim 4, **CHARACTERIZED IN THAT** the aqueous medium of step (ii) is an aqueous continental medium or a saline medium, wherein the aqueous medium may be continental water, also known as aquifer water or water from natural watercourses, distilled water, non-desalinated seawater, or a saline discard medium derived from lithium production processes or seawater desalination processes, the latter being capable of reaching chloride concentrations between 45-85 g/L from sodium salts.

6. The method for obtaining the BioReagent or leaching solution according to claim 4, **CHARACTERIZED IN THAT** in step (e) the sulfur-oxidizing and/or iron-oxidizing bioleaching microorganisms are selected from Ferroplasma acidiphilum, Leptospirillum ferrooxidans, Acidianus infernus, Acidithiobacillus ferrooxidans, and Acidithiobacillus thiooxidans.

7. Use of the BioReagent according to claim 1, **CHARACTERIZED IN THAT** it is used in a hydrometallurgical process consisting of agitated leaching of minerals, metal concentrates, mining tailings, spent ore residues, metallurgical slags, and Waste Electrical and Electronic Equipment (WEEE).

8. Use of the BioReagent according to claim 1, **CHARACTERIZED IN THAT** it is used for recovering metals from Waste Electrical and Electronic Equipment (WEEE) selected from computing equipment, printed circuit boards (PCBs), PCBs from computers and mobile phones, televisions and monitors, displays, photovoltaic panels, solar energy systems, and others such as desktop and laptop computers; tablets and similar electronic devices; lamps; kitchen appliances including microwaves and toasters; washing machines and dryers; refrigerators and freezers; air conditioning and heating units; audio equipment and sound systems; cameras and camcorders; video game consoles and accessories; storage devices including hard drives and USB memory devices; printers and scanners; electronic exercise equipment; electronic medical devices; electrical and electronic tools; surveillance and security equipment; communication equipment including routers and modems; electronic toys and entertainment devices; watches and wearable devices; electronic office equipment including photocopiers and fax machines; electronic lighting equipment including LED lamps; industrial control and automation equipment; general consumer electronics; large rechargeable batteries including those used in electric vehicles; cryptocurrency mining equipment including ASICs and mining rigs; specialized laboratory and medical equipment including magnetic resonance imaging and tomography systems; virtual and augmented reality devices including VR/AR headsets and controllers; home automation equipment including sensors and control systems; industrial and commercial Internet of Things (IoT) devices; satellite communication equipment and parabolic antennas; tracking devices including GPS units and locators; 3D printing equipment and 3D scanners; small-scale wind energy systems; hydroponic cultivation equipment and agricultural control systems; advanced scientific research equipment including particle accelerators; thermal and geothermal energy generation systems; and alternative entertainment devices including drones and flight simulators.

9. Use of the BioReagent according to claim 1, **CHARACTERIZED IN THAT** it is used for recovering metals such as gold, silver, palladium, and copper from Waste Electrical and Electronic Equipment (WEEE).

10. A process for recovering metals from Waste Electrical and Electronic Equipment (WEEE), **CHARACTERIZED IN THAT** it comprises the following steps:
i. crushing the WEEE to obtain particles having an F80 between 3 and 25 mm;
ii. loading the crushed WEEE from step (i) up to 35% solids into a reactor, flooding with the BioReagent or leaching solution according to claim 1, agitating the mixture at a speed ranging from 250 to 800 rpm, homogenizing and maintaining a temperature between 35 and 85 °C, with forced aeration at a rate of 0.08 to 0.25 L air/min/L slurry, at atmospheric pressure (1 atm), for a period of 5 to 15 days;
iii. replacing the loaded leaching solution with fresh solution and incorporating fresh feed material into the process as a function of the pH and/or Eh of the solution, monitored at intervals of 8 to 24 hours;
iv. terminating the leaching process by stopping agitation and forced aeration, thereby allowing the solids contained in the slurry to settle for a period of 40 to 60 minutes;
v. separating and discharging the copper-enriched solution (PLS) from the slurry obtained in step (iv);
vi. discharging the pulp concentrated in solids obtained in step (v), subjecting it to solid-liquid separation by filtration, followed by drying at a temperature between 60 and 90 °C for 12 to 30 hours;
vii. sieving the filtered and dried solid material obtained in step (vi) using a mesh having an aperture size between 250 and 1000 µm, thereby obtaining a fine product and a coarse spent leach residue.

11. The process for recovering metals according to claim 10, **CHARACTERIZED IN THAT** step (ii) is carried out in batch or continuous mode.

12. The process for recovering metals according to claim 10, **CHARACTERIZED IN THAT** the copper-enriched solution obtained in step (v) is pumped to at least one storage unit.
